# EUROPEAN PATENT APPLICATION

(11) **EP 3 095 854 A1**
(43) Date of publication of application: **23.11.2016**
(21) Application number: 15737796.1
(22) Date of filing: 15.01.2015
(51) Int. Cl.: C12N 1/04, A23P 10/30, B01J 13/02, B82Y 5/00

(54) **METHOD FOR PROTECTING BIOLOGICAL MATERIAL AND THERMOLABILE COMPOUNDS FOR POSSIBLE INDUSTRIAL USES**

(30) Priority: 15.01.2014 ES 201430034
(71) Applicant: Consejo Superior de Investigaciones Cientificas (CSIC), 28006 Madrid (ES)
(72) Inventor: LAGARÓN CABELLO, José María, 46980 Paterna (Valencia) (ES); PÉREZ MASÍA, Rocío, 46980 Paterna (Valencia) (ES); LÓPEZ RUBIO, Amparo, 46980 Paterna (Valencia) (ES)
(74) Representative: Pons
(86) International application number: PCT/ES2015/070020
(87) International publication number: WO 2015/107246

(57) **Abstract**

The invention relates to a method for protecting biological material in general and thermolabile substances, particularly useful in microorganisms and viruses and any other biological material, as well as in any substance, derived or otherwise, that requires protection to extend the useful life thereof or broaden the scope of use thereof, by electrohydrodynamic or aerodynamic processing.

Said processing makes it possible to extend the useful life of the product in a unique manner, since exposing the substance to be protected to a temperature is not involved. In addition, depending on the features and variables used during the coating process, encapsulation makes it possible to maintain the viability of the product against stress conditions such as temperature, relative humidity or pH among others, offering effective protection during the subsequent preparation, processing and storage thereof, and even, for example, during the passage thereof through the gastrointestinal tract in the case of probiotic microorganisms.

## Description

### SECTOR AND OBJECT OF THE INVENTION

Food and pharmaceutical sectors. Materials designed to stabilise microorganisms, viruses and biological material, as well as high-added-value thermolabile and bioactive compounds or substances.

The object of the present invention is a process for protecting biological material in general and thermolabile substances, specifically applicable to microorganisms and viruses and any other biological material, as well as to any substance, whether a derivative or not, that requires protection in order to prolong its useful life or expand its field of application, by means of electrohydrodynamic or aerodynamic processing. Specifically, micro-, submicro- and nanoparticles of different polymers are obtained by means of electrohydrodynamic or aerodynamic processing, also known as electrospinning, electrospraying, solution blow spinning and solution blow spraying. These micro-, submicro- and nanoparticles are directly processed on preparations of microorganisms (lyophilisates or preparations dehydrated by any other preservation method), viruses, biological material or thermolabile substances, such that, due to the high surface-volume ratio of the morphology obtained, they become adhered, to form an effective protective coating or capsule that coats all the interstices of the product, and is therefore more efficient than other methods or processes in preserving the viability (microorganism count or number of colony-forming units, CFU) or stability of the product. This process makes it possible to prolong the useful life of the product in a unique manner, since it does not involve exposing the substance to be protected to temperature. Moreover, depending on the characteristics or variables used during the coating process, encapsulation makes it possible to maintain the viability of the product under stress conditions, such as temperature, relative humidity or pH, among others, by providing it with effective protection during the subsequent preparation, processing and storage thereof, and even, for example, during passage through the gastrointestinal tract in the case of probiotic microorganisms.

### PRIOR ART

Within the field of probiotics, there is an increasing demand for functional products based on microorganisms that are beneficial for health. However, it is necessary for these microorganisms to be in the product in quantities greater than 10⁷, ideally 10⁹ colony-forming units (CFU/ml), at the time of consumption, in order for the effect to be efficient [Gerez, C.L., Font de Valdez, G., Gigante, M.L., Grosso, C.R.F. (2012). Whey protein coating bead improves the survival of the probiotic Lactobacillus rhamnosus CRL 1505 to low pH. Letters in Applied Microbiology 54 (6), pp. 552-556]. Since most of these microorganisms are sensitive to environmental conditions (humidity, oxygen concentration, light, etc.), it is important to develop strategies that protect them from the environment in order to preserve them. Thus, numerous processes are being studied for the development of structures capable of providing the necessary conditions for an optimal metabolism of the microorganisms, whilst protecting them from their surroundings. Some of the techniques used for the immobilisation and protection of microorganisms include cellular adsorption on solid supports [Rezaee, A., Godini, H., Bakhtou, H. (2008). Microbial cellulose as support material for the immobilization of denitrifying bacteria. Environmental Engineering and Management Journal, 7 (5), pp. 589-594], [4], and flocculation [5] [D. Yin, D., Liu, C., Li, F., Ge, X., Bai, F. (2011). Development of observed kinetic model for self-flocculating yeast. Huagong Xuebao/CIESC Journal, 62 (11), pp. 3149-3155]. Both techniques require that the microorganisms be capable of naturally adhering to the supports (capacity to form biofilms) or aggregating in order to flocculate, respectively. Moreover, other strategies such as encapsulation have been studied [Rathore, S., Desai, P.M., Liew, C.V., Chan, L.W., Heng, P.W.S. (2013). Microencapsulation of microbial cells. Journal of Food Engineering 116 (2), pp. 369-381]. The most common of these include encapsulation by extrusion, coacervation, spray-drying and emulsification [de Vos, P., Faas, M.M., Spasojevic, M. Sikkema, J. (2010). Encapsulation for preservation of functionality and targeted delivery of bioactive food components. International Dairy Journal, 20 (4), pp. 292-302]. However, these technologies require heating of the solutions or the use of organic agents in at least one of the production stages, which may damage or destroy the encapsulated microorganisms. Therefore, it would be desirable to have new technologies available that do not involve aggressive conditions (both in regards to the temperature and the solvents used) and which lead to small particle sizes. The electrohydrodynamic processing technique, which comprises processes called high-voltage electrospinning and electrospraying, is a simple, highly versatile method for obtaining fibres and/or capsules through the action of an external electric field that is applied between two electrodes and whereto the polymer solution is subjected. The properties of the structures obtained by means of electrohydrodynamic processing include their nanometric size and the fact that they do not require using temperature; consequently, the technique has already been proposed for the encapsulation of probiotics with good results [Lopez-Rubio, A., Sanchez, E., Sanz, Y., and Lagaron, J.M. (2009). Encapsulation of living bifidobacteria in ultrathin PVOH electrospun fibers. Biomacromolecules 10, 2823-2829]. On the other hand, the aerodynamic processing technique, which comprises solution blow spinning and spraying, involves applying pressure differences in order to accelerate a fluid and thus obtain the different polymer structures. This technology has been used for the encapsulation of active agents [Oliveira, J.E., Medeiros, E.S., Cardozo, L., Voll, F., Madureira, E.H., Mattoso, L.H.C., Assis, O.B.G. (2013). Development of poly (lactic acid) nanostructured membranes for the controlled delivery of progesterone to livestock animals. Materials Science and Engineering C 33 (2), pp. 844-849], as well as probiotics [Spanish patent application P201131048]. In any case, direct encapsulation disperses and confines the microorganisms in a closed space, requires the dissolution thereof, stability and compatibility with the media and the solvents, and, moreover, that the materials used to form the capsules correctly match the metabolic needs of the encapsulated microorganisms (adequate mechanical, solvent and permeability properties in relation to nutrients, gases and metabolites) in order for their viability to be maintained in time. Moreover, all these encapsulation technologies require that the microorganisms be subjected to various conditions that may affect their viability (temperature, organic solvents, high voltage or pressure differences). Therefore, the microorganisms are always affected to a greater or lesser extent and, even if the long-term viability is maintained, the initial count usually decreases. In this regard, the coating protection technique by means of electrohydrodynamic or aerodynamic processing of the present invention provides very efficient protection of the microorganism preparations, since protective micro-, submicro- and nanostructures are obtained which are adhered thereto, thereby protecting them against external conditions and, consequently, maintaining their viability for a longer period of time. Moreover, it avoids any type of direct processing of the material, for which reason the initial viability is maintained, which has great industrial relevance. On the other hand, when using this technique, the microorganisms are not confined in a rigid matrix, but simply enveloped in an encapsulation coating; for this reason, it does not interfere with the metabolism.

### DESCRIPTION OF THE INVENTION

The present invention consists of a methodology for protecting microorganisms, viruses, biological material in general and any thermolabile substance that requires protection in order to prolong its useful life or expand its field of application, by means of a coating composed of polymer micro-, submicro- or nanostructures obtained by means of electrohydrodynamic or aerodynamic processing, which adhere to the microorganisms, thereby generating a protective encapsulation effect. In this way, the thermolabile compounds and biological materials to be encapsulated are not subjected to any type of processing, and are protected and isolated from the environment. To this end, micro-, submicro or nanoparticles are obtained by means of electrospinning/electrospraying, more preferably by means of electrospraying, or blow spinning/spraying, more preferably by means of blow spraying, from synthetic or natural polymers (biopolymers), preferably soluble in water and primarily biopolymers, since they are usually more beneficial for the microorganisms.

The process for protecting biological material and thermolabile compounds of industrial interest comprises the following steps:
- preparing polymer solutions which form micro-, submicro- and nanoparticles that compose coating materials
- coating the biological material or thermolabile compounds with the materials prepared in the preceding step, the coating of the biological material or thermolabile compounds being performed by means of electrohydrodynamic or aerodynamic processing.

The biological material preferably is microorganisms or viruses, and, in particular, the microorganisms are selected from *Lactobacillus, Bifidobacterium, Cyanobacterium, Rhodobacterales, Saccharomyces* or any other microorganism that requires protection.

The thermolabile compounds are enzymes, vitamins, essential elements, or any derivative molecule or compound.

The coating materials are selected from proteins, oligosaccharides, polysaccharides, lipids, polymers and combinations thereof.

The polymers are particularly selected from water-soluble polymers, alcohols and mixtures of these, such as polyethylene oxide, ethylene-vinyl alcohol copolymers, polyvinyl alcohol, polyvinyl pyrrolidone and combinations thereof.

The proteins are particularly selected from zein and whey protein.

The polysaccharides are particularly selected from dextran, maltodextrin and starch, and any combination or preparation thereof, such as the commercial preparation based on a resistant starch called fibersol.

The solvent used for the preparation of the polymer solutions is selected from water, mixtures of alcohols and water, and other organic solvents, preferably water or mixtures of alcohol and water.

In the step of the polymer solutions preparation, processing aiding substances may be added which facilitate the formation of the micro-, submicro- and nanoparticles, and which are selected from plasticisers, surface active agents, emulsifiers, surfactants, antioxidants or any combinations thereof, preferably sorbitan monolaurate (commercially known as span-20). Homogenisation treatments by stirring, tipping or ultrasound may also be included.

In a preferred embodiment, the electrohydrodynamic or aerodynamic processing is electrospraying or blow spraying in at least one step. Optionally, the electrohydrodynamic or aerodynamic processing is performed in several steps, with a homogenisation and/or sieving treatment between them.

The electrohydrodynamic or aerodynamic processing may be directly performed on the biological material or thermolabile compounds previously dehydrated by means of lyophilisation or another non-electrohydrodynamic or non-aerodynamic process, or without prior dehydration, the dehydration and the application of the coating taking place in successive processings by electrohydrodynamic or aerodynamic processing.

In the event that it is necessary, prior to or following the coating step, a sieving treatment is performed in order to control the particle size, preferably mechanical sieving.

### DETAILED DESCRIPTION OF THE INVENTION

Below it is described in detail the methodology for obtaining microorganism coatings by means of electrohydrodynamic or aerodynamic processing. The objective of these coatings is to stabilise and maintain the viability of various microorganisms or thermolabile substances with industrial applications such that they may, for example, be maintained at high concentrations at the time of their application (consumption, sensors, water treatment, etc.).

The first step consists of preparing the polymer solutions that form the micro-, submicro- and nanoparticles which make up the coatings. During this stage, other substances (such as plasticisers, surface active agents, emulsifiers, surfactants, antioxidants, processing aids in general or any combinations thereof) may be added in order to facilitate the formation of the structures.

The surfactants are preferably selected, without being limited thereto, from different types of compounds commonly called span, different types of tween and lecithin, and, more preferably, the one commercially called span-20 whose composition is sorbitan monolaurate.

This step also includes a homogenisation treatment by stirring and/or ultrasound. The stirring may be vigorous in order to favour the dispersion of the additives in the polymer matrix.

The second step consists of coating the microorganisms by electrohydrodynamic or aerodynamic processing. This process may be directly performed on the formulated commercial product, dehydrated by methods such as lyophilisation or electrohydrodynamic or aerohydrodynamic processing, or may be obtained *in situ* by electrohydrodynamic or aerodynamic processing, or by any other process, without limitation, prior to the coating.

The electrohydrodynamic processing, which comprises electrospinning and electrospraying, is a technology based on the application of high electric fields in order to produce electrically charged fluids from viscoelastic polymer solutions which, when dried, produce micro- and nanofibres, or micro- and nanocapsules, respectively. The electrospinning and electrospraying equipment is composed of a power supply that supplies a current, a pump where the reservoirs with the solutions and one or several conductive propellants, typically needles, made of a conductive material are placed. The needles are connected to the reservoirs and channelled towards the collector, where the dry material will be colected. In the case of aerodynamic processing, which comprises blow spraying and blow spinning, a pump is used, where the reservoirs with the solutions are placed. These reservoirs are connected to a nozzle through which a pressurised gas flow is also made to pass. The nozzle is channelled towards the collector, where the material is collected. Thus, the microorganism preparation is placed on the collector and the protective particles, which have a controlled size, are directly spinned or sprayed on them to form a coating that provides stability to the product during the processing, post-processing and storage thereof, and even when it is applied.

In the present invention, the electrohydrodynamic processing preferably uses electrospraying and the aerodynamic processing preferably uses blow spraying.

In all cases, a gravity, mechanical or any other type of sieving treatment may be performed on the microorganism preparation prior to and following the coating, in order to control the particle size.

The coating process may be performed in one or several steps, depending on the protection needs of the material, and with one or several materials, and may involve tipping and homogenisation processes for the coating using any existing industrial means, in order to increase the efficacy of the coating process.

The materials used for the coatings obtained in the manner described above comprise proteins, oligosaccharides, polysaccharides, lipids, other water-soluble polymers, such as, without being limited thereto, polyvinyl alcohol, polyethylene oxide or polyvinyl pyrrolidone, and alcohol-water mixtures, such as, without being limited thereto, ethylene-vinyl alcohol copolymers and any commercial preparation thereof or mixture of the above. Preferably, proteins, oligosaccharides, polysaccharides and lipids will be used, due to their sustainable character and their greater stability and compatibility with the microorganisms.

The proteins will be preferably selected, without being limited thereto, from animal, vegetable and microbial proteins, such as, for example, whey proteins, caseins, polypeptides, whether natural or obtained by the genetic modification of microorganisms, collagen, soy protein and zein protein, and, more preferably, the zein and the whey protein.

The oligosaccharides will be preferably selected, without being limited thereto, from lactose, sucrose, maltose and fructo-oligosaccharides, and, more preferably, fructo-oligosaccharides.

The polysaccharides will preferably be selected, without being limited thereto, from alginate, pectins, chitosan, gums, carrageenans, starch, dextran, maltodextrin, cellulose, glycogen, chitin and, more preferably, starch, dextran, maltodextrin and any preparation thereof, such as fibersol or similar preparations.

The lipids will be preferably selected, without being limited thereto, from fatty acids.

In regards to the solvents, water is the preferred one, but, since the technique eliminates the solvent very efficiently, the process of the invention is compatible with any type of solvent, whether or not it is compatible with the product to be protected, this being one of the advantages of this process. In regards to the microorganisms, in the case of probiotics, both facultative aerobic bacteria belonging to the genus *Lactobacillus,* for example *Lactobacillus plantarum,* and strict anaerobic bacteria of the genus *Bifidobacterium,* for example *Bifidobacterium longum or Bifidobacterium longum* subsp. *infantis,* or the CECT 4552 strain of *Bifidobacterium longum* subsp. *infantis,* have been tested. Fungi of the genus *Saccharomyces* may also be used in this application. In the case of water treatment or sensor applications, the preferred families are *Cyanobacterium* or *Rhodobacterales.*

Throughout the description and the claims, the word "comprises" and variants thereof are not intended to exclude other technical characteristics, additives, components or steps. For persons skilled in the art, other objects, advantages and characteristics of the invention will arise, partly from the description and partly from the implementation of the invention. The following examples and figures are provided for illustrative purposes, and are not intended to limit the scope of the present invention.

### BRIEF DESCRIPTION OF THE FIGURES

Figure 1. A Scanning Electron Microscopy (SEM) image of electrosprayed structures of starch in aqueous solution.
Figure 2. Example of an optical microscopy image of crystals of the *Lactobacillus plantarum* lyophilisate coated with the polymer structures (alginates, pectins and modified starch) obtained by electrospraying.
Figure 3. Counts of the coated lyophilisate vs. the non-coated lyophilisate with time, stored at 37°C.
Figure 4. Counts of the coated lyophilisate vs. the non-coated lyophilisate with time, stored at 53% relative humidity (RH).
Figure 5. A Scanning Electron Microscopy (SEM) image of the crystals of the *Lactobacillus plantarum* lyophilisate.
Figure 6. A Scanning Electron Microscopy (SEM) image of the crystals of the *Lactobacillus plantarum* lyophilisate coated with whey proteins by means of the method of the present invention.
Figure 7. A Scanning Electron Microscopy (SEM) image of capsules of zein coating the crystals of the *Lactobacillus plantarum* lyophilisate.
Figure 8. A Scanning Electron Microscopy (SEM) image of capsules of whey protein concentrate coating the crystals of the *Bifidobacterium longum* subsp. *infantis* (CECT 4552) lyophilisate.
Figure 9. Counts of the coated lyophilisate vs. the non-coated lyophilisate with time, stored at: (A) 37°C, (B) 53% RH, and (C) 26% RH.

### EMBODIMENT OF THE INVENTION

### Example 1.

### Obtainment of the nanostructured coating from a polysaccharide.

This example describes a typical process for obtaining coatings based on a polysaccharide using the electrospraying technique.

In a first step, the polysaccharide solution (of the commercial brand fibersol) is prepared in distilled water. The polysaccharide concentration used is 40% by weight with respect to the volume of the solvent. The solution is stirred at room temperature until a homogeneous solution is obtained.

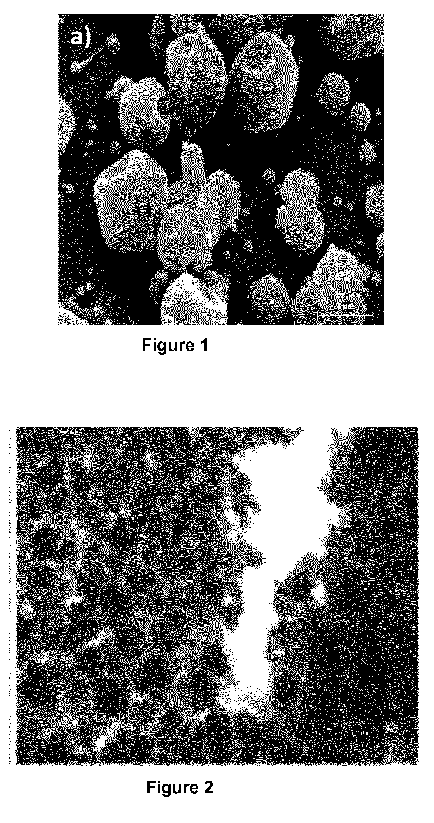

Once the solution has been obtained, it is used to generate the micro- and nanocapsules by means of the electrospraying technique, using commercial equipment of the brand Fluidnatek, from Bioinicia, S.L., Paterna (Valencia). The solution is introduced into 5-ml syringes connected to a stainless steel needle with a diameter of 0.9 mm through teflon tubing. The needle is connected to an electrode which, in turn, is connected to a 0-30 kV power supply. A voltage ranging between 15-20 kV is applied and the solution is pumped through said needle at a flow rate of 0.1 ml/h. The counter electrode is connected to a stainless steel plate (collector), where the lyophilised probiotic is placed. The distance between the needle and the collector is about 15 cm. The process is performed at room temperature. In this way, the micro- and nanocapsules shown in Figure 1 are obtained, which adhere to the lyophilisate particles.

The sizes of the capsules obtained in this manner range between 50 nm and 3 microns (i.e. capsules with sizes lower than 100 nm).

### Example 2.

### Obtainment of a nanostructured coating from a mixture of biopolymers (alginates, pectins and modified starch) and application thereof on the Lactobacillus plantarum lyophilisate.

The first step consists of preparing the solution that forms the nanoparticle coating. To this end, the mixture of alginate, pectin and modified starch is dissolved at a concentration of 3.1% and 10% by weight with respect to the volume of the solvent (distilled water), respectively. The mixture is kept under stirring until the components are completely dissolved. The final solution is introduced into a 5 ml syringe, in order to subsequently make it pass through the electrohydrodynamic processing equipment.

In the second place, a fine, homogeneously distributed deposition of the lyophilisate (approx. 5 x 10¹⁰ cfu/gram) is performed by means of a sieve, and distributed on the collector of the electrohydrodynamic processing equipment. Subsequently, the solution is electrosprayed on the lyophilisate. The equipment is composed of a power supply that supplies a current ranging between 0 and 30 kV, a digital pump where the syringe is placed, and which makes it possible to control the flow rate of the solution, and a stainless steel needle. The needle is connected to the syringe through a teflon tube and placed perpendicular to the collector. In this example, the electrospinning conditions were the following: flow rate of the solution of 0.15 ml/h; 14 kV of current; 10 cm distance between the needle and the collector. Following a controlled electrospraying time, the material is tipped and electrosprayed once again, in order to ensure complete coating of the entire lyophilisate. The material collected under these conditions consists of a nanostructured system composed of spherical capsules of the mixture of alginates, pectins and starch that coat the lyophilisate crystals (as shown in Figure 2).

### Example 3.

### Obtainment of a nanostructured coating from Polyethylene oxide (PEO) and application thereof on the Lactobacillus plantarum lyophilisate.

The first step consists of preparing the solution that forms the nanoparticle coating. To this end, the PEO is dissolved at a concentration of 4% by weight with respect to the volume of the solvent (distilled water). The solution is kept under stirring until the PEO is completely dissolved. The final solution is introduced into a 5-ml syringe in order to subsequently make it pass through the electrohydrodynamic processing equipment.

In the second place, the lyophilisate deposition is carried out and homogeneously distributed on the collector of the electrohydrodynamic processing equipment. Subsequently, the solution is electrosprayed on the lyophilisate. The equipment is composed of a power supply that supplies between 0 and 30 kV of current, a digital pump where the syringe is placed and which makes it possible to control the flow rate of the solution, and a stainless steel needle. The needle is connected to the syringe through a teflon tube and placed perpendicular to the collector. In this example, the electrospraying conditions were the following: flow rate of the solution of 0.15 ml/h; 14 kV of current; 10-cm distance between the needle and the collector. Following a controlled electrospraying time, the material is tipped and electrosprayed once again, in order to ensure complete coating of the entire lyophilisate. The material collected under these conditions is a nanostructured system composed of PEO fibres that coat the lyophilisate crystals.

### Example 4.

### Obtainment of a nanostructured coating from a mixture of Polyvinyl pyrrolidone (PVP) and polyvinyl alcohol (PVOH) and application thereof on the Lactobacillus plantarum lyophilisate.

The first step consists of preparing the solution that forms the nanoparticle coating. To this end, the PVP and the PVOH are dissolved at a concentration of 20% and 4% by weight with respect to the volume of the solvent (distilled water), respectively. The solution is kept under stirring until all the components are completely dissolved. The final solution is introduced into a 5-ml syringe in order to subsequently make it pass through the electrospraying equipment.

In the second place, the lyophilisate is deposited and homogeneously distributed on the collector of the electrospraying equipment. Subsequently, the solution is electrosprayed on the lyophilisate. The electrospraying equipment is composed of a power supply that supplies between 0 and 30 kV of current, a digital pump where the syringe is placed, and which makes it possible to control the flow rate of the solution, and a stainless steel needle. The needle is connected to the syringe through a teflon tube and placed perpendicular to the collector. In this example, the processing conditions were the following: flow rate of the solution of 0.15 ml/h; 14 kV of current; 10-cm distance between the needle and the collector. Following a controlled electrospraying time, the material is tipped and electrosprayed once again, in order to ensure complete coating of the entire lyophilisate. The material collected under these conditions is a nanostructured system composed of PVP and PVOH capsules that coat the lyophilisate crystals.

### Example 5.

### Encapsulation of probiotics (Lactobacillus plantarum) from a whey protein concentrate using different electrohydrodynamic processes and comparison of the initial viability of the different materials.

The first step consists of preparing the solutions that form the encapsulates and the coating. To this end, the whey protein concentrate is dissolved at a concentration of 20% by weight with respect to the volume of the solvent (skim milk). Moreover, a surfactant (Span-20) is added, at a concentration of 20% with respect to the weight of the protein, in order to improve the processing. The mixture is kept under stirring until the components are completely dissolved.

In the second place, the probiotic microorganism is added according to the processing that is to be performed. For uniaxial electrospraying, the lyophilisate is added to the solution at a concentration of up to 50% by weight with respect to the weight of the polymer. In the case of coaxial electrospraying, a lyophilisate solution is prepared that is 50% by weight with respect to the volume of the solvent (saline solution). Finally, for the coating, the lyophilisate is deposited and homogeneously distributed on the collector of the electrospinning equipment. Subsequently, the solutions are electrosprayed. In the case of the coating, following a controlled electrospraying time, the material is tipped and electrosprayed once again, in order to ensure complete coating of the entire lyophilisate. The equipment used for the uniaxial process and the coating is composed of a power supply that supplies between 0 and 30 kV of current, a digital pump where the syringe is placed and which makes it possible to control the flow rate of the solution, and a stainless steel needle. The needle is connected to the syringe through a teflon tube and placed perpendicular to the collector. In the case of coaxial electrospraying, the equipment is made up of the same elements as above, but it additionally has another digital pump to place the syringe with the lyophilisate and two concentric needles, through which the polymer solution (through the outer needle) and the lyophilisate solution (through the inner needle) will circulate.

The electrospraying conditions were the following: flow rate of the solution of 0.15 ml/h; 14 kV of current; 7 cm distance between the needle and the collector in the case of the uniaxial processing and the coating. In the case of the coaxial processing, they were: 0.16 ml/h for the protein solution, 0.08 ml/h for the lyophilisate solution, 20 kV of current and 7 cm distance between the needle and the collector.

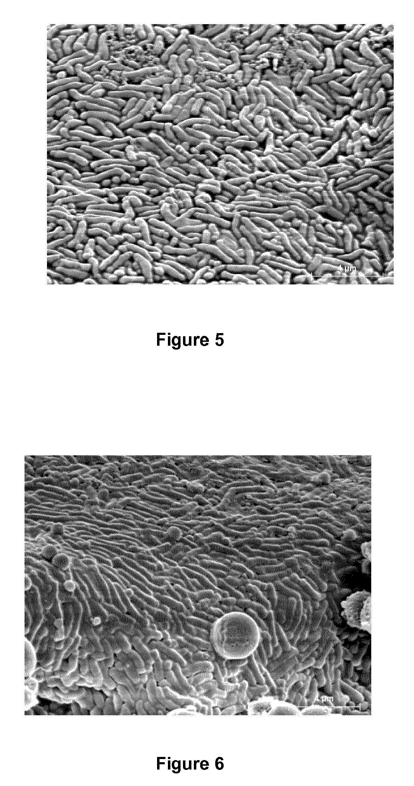

Finally, the viability of the microorganisms of the different materials obtained was measured, observing that the coating method provided higher counts, similar to the counts for the starting, unprotected lyophilisate, than for the lyophilisate encapsulated by means of the uniaxial and coaxial methods (Table 1). Figure 6 shows the electron microscopy image of the coated lyophilisate as compared to the image of the uncoated lyophilisate crystals (Figure 5).

**Table 1**

| | Coaxial encapsulation | Uniaxial encapsulation | Coating |
|---|---|---|---|
| Counts (CFU/g) | 2.3 x 10⁸ | 2.1 x 10⁸ | 8.2 x 10⁹ |

### Example 6.

### Obtainment of submicrometric encapsulation structures from a maltodextrin using the blow spinning/spraying technique.

This example explains in detail the process for obtaining submicrometric capsules by means of the blow spinning/spraying technique. In the first place, a solution of maltodextrin in water is prepared, using a polysaccharide concentration of 40% by weight with respect to the volume, and stirring at room temperature until a homogeneous solution is obtained.

The solution was introduced into a 5-ml syringe located in a syringe pump and connected to an inner stainless steel needle with a diameter of 0.9 mm through teflon tubing. This needle was mounted in a coaxial configuration, with the nitrogen gas, pressurised at high speed (230-250 m/s), flowing through the outer needle. The nitrogen flow, coaxially pumped through the outer needle, accelerates and spins the polymer solution that flows through the inner needle and contributes to the formation of the encapsulation structures. The flow rate of the maltodextrin solution was 0.5 ml/h. The pressure of the nitrogen gas in the bottle was 20-30 bar. The solidified structures generated were collected in a collector located at a distance of about 18-20 cm.

### Example 7.

### Viability results for Lactobacillus plantarum following application of the nanostructured coating obtained from several polymer formulations.

This example shows the viability results for *L. plantarum* obtained immediately after coating the microorganisms by means of electrohydrodynamic processing. In the first place, the different polymer solutions with the corresponding proportions and additives for each material, were prepared. These formulations were made to pass through the electrospraying equipment in order to obtain the nanostructured coating. Subsequently, a fixed quantity (0.2 g) of lyophilisate was homogeneously deposited on the collector where the nanostructured material was collected. Finally, the solutions are electrosprayed on the lyophilisate. Following a controlled electrospraying time, the material is tipped and electrosprayed once again, in order to ensure complete coating of the entire lyophilisate. The equipment is composed of a power supply that supplies between 0 and 30 kV of current, a digital pump where the syringe is placed, and which makes it possible to control the flow rate of the solution, and a stainless steel needle. The needle is connected to the syringe through a teflon tube and placed perpendicular to the collector. The electrospraying conditions were adjusted according to the different polymers used. The material collected under these conditions consists of a nanostructured system composed of polymer capsules that coat the lyophilisate crystals. Immediately after collecting it, the material was seeded in the optimal medium for growth of the bacteria and the microorganism counts were performed. The viability results obtained are shown in Table 2.

**Table 2.**

| **Coating-forming materials** | **Initial viability (CFU/g)** |
|---|---|
| Alginate | 1.10E+10 |
| Alginate + Pectins + Fibersol + Span-20 | 5.70E+09 |
| Alginates + Fibersol + Span-20 | 4.60E+09 |
| Starch | 2.30E+09 |
| Starch + Polyvinyl alcohol (PVOH) | 1.20E+09 |
| Starch + Span-20 | 1.20E+10 |
| Dextran | 3.50E+09 |
| Dextran + Span-20 | 1.20E+10 |
| Maltodextrin + PVOH | 1.30E+09 |
| Maltodextrin + Span-20 | 2.40E+09 |
| Polyethylene oxide (PEO) | 1.30E+10 |
| Pullulan | 1.20E+10 |
| Pullulan + PVOH | 1.10E+10 |
| PVOH | 2.40E+10 |
| Polyvinyl pyrrolidone (PVP) + PVOH | 3.40E+10 |
| PVP + Span-20 | 2.30E+10 |
| Whey protein concentrate (WPC) | 6.20E+09 |
| WPC + PVOH | 3.10E+09 |
| WPC + Span-20 | 1.80E+09 |
| Zein | 1.10E+10 |

### Example 8.

### Coating of Lactobacillus plantarum with resistant starch by electrohydrodynamic processing and study of the viability with time under temperature conditions.

The first step consists of preparing the solution that forms the nanoparticle coating. To this end, the resistant starch is dissolved at a concentration of 20% by weight with respect to the volume of the solvent (distilled water). Moreover, 2% of the surfactant Span 20 is added in order to facilitate the electrospraying process. The solution is kept under stirring until all the components are completely dissolved. The final solution is introduced into a 5-ml syringe in order to subsequently make it pass through the equipment.

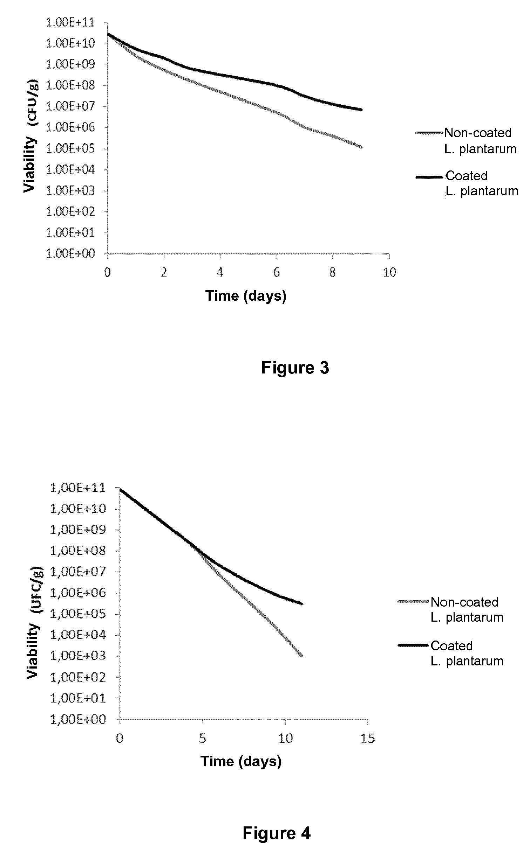

In the second place, the lyophilisate is deposited and homogeneously distributed on the collector of the electrospinning equipment by means of a sieve. Subsequently, the solution is electrosprayed on the lyophilisate. Following a controlled electrospraying time, the material is tipped and electrosprayed once again in order to ensure complete coating of the entire lyophilisate. The electrospraying equipment is composed of a power supply that supplies between 0 and 30 kV of current, a digital pump where the syringe is placed, and which makes it possible to control the flow rate of the solution, and a stainless steel needle. The needle is connected to the syringe through a teflon tube and placed perpendicular to the collector. In this example, the electrospraying conditions were the following: flow rate of the solution of 0.15 ml/h; 14 kV of current; 10 cm distance between the needle and the collector. The material collected under these conditions is a nanostructured system composed of resistant starch capsules that coat the lyophilisate crystals. This material was stored jointly with non-coated lyophilisate at 37°C in order to perform an accelerated viability study of the microorganisms. Figure 3 shows the counts of the 2 stored samples (coated and non-coated lyophilisate). It may be observed that the initial counts are similar in both cases. However, as the storage time under these conditions increases, the viability of the non-coated lyophilisate decreases more rapidly than that of the protected lyophilisate.

### Example 9.

### Coating of Lactobacillus plantarum with zein by electrohydrodynamic processing and study of the viability with time under humidity conditions.

The first step consists of preparing the solution that forms the nanoparticle coating. To this end, the zein is dissolved at a concentration of 12% by weight with respect to the volume of the solvent (a mixture of alcohol and water at a 15:85 proportion, respectively). The solution is kept under stirring until the zein is completely dissolved. The final solution is introduced into a 5 ml syringe in order to subsequently make it pass through the electrospraying equipment.

In the second place, the lyophilisate is deposited and homogeneously distributed on the collector of the electrohydrodynamic processing equipment.

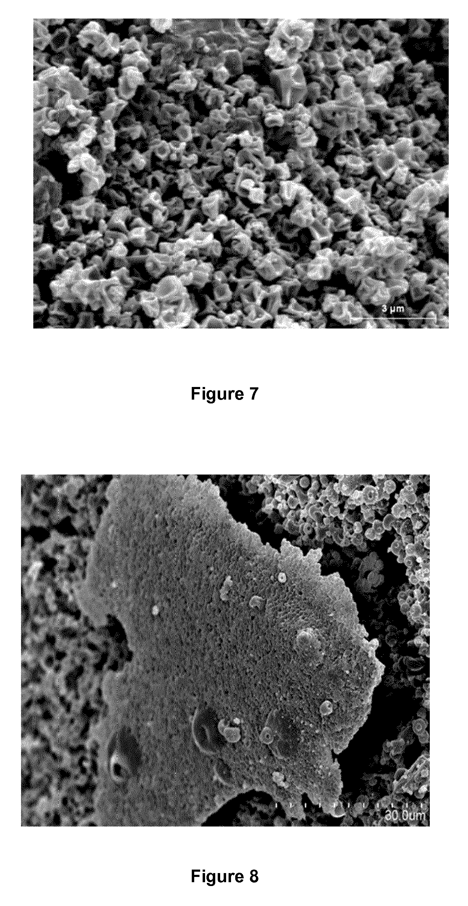

Subsequently, the solution is electrosprayed on the lyophilisate. Following a controlled electrospraying time, the material is tipped and electrosprayed once again, in order to ensure complete coating of the entire lyophilisate. The equipment is composed of a power supply that supplies between 0 and 30 kV of current, a digital pump where the syringe is placed, and which makes it possible to control the flow rate of the solution, and a stainless steel needle. The needle is connected to the syringe through a teflon tube and placed perpendicular to the collector. In this example, the electrospraying conditions were the following: flow rate of the solution of 0.30 ml/h; 12 kV of current; 10 cm distance between the needle and the collector. The material collected under these conditions is a nanostructured system composed of zein capsules that coat the lyophilisate crystals, as observed in Figure 7, as compared to the image of the crystals of the non-coated lyophilisate (Figure 5). This material was stored with non-coated lyophilisate at 53% relative humidity in order to perform an accelerated study of the viability of the microorganisms. Figure 4 shows the counts for the 2 stored samples (coated and non-coated lyophilisate). It may be observed that, at first, the viability of the coated lyophilisate presents a sharper decrease; however, soon thereafter it is stabilised, whereas the unprotected lyophilisate decreases exponentially.

### Example 10.

### Coating and encapsulation of β-carotene with zein by electrohydrodynamic processing and comparative study of the stability of the antioxidant under ultraviolet light (UV).

The first step consists of preparing the solutions that form the nanoparticle coating and the encapsulates. For the coating, the zein is dissolved at a concentration of 12% by weight with respect to the volume of the solvent (a mixture of alcohol and water at a proportion of 15:85, respectively). In order to obtain the encapsulated β-carotene, a 33% solution of zein by weight with respect to the volume of the solvent (a mixture of alcohol and water at a proportion of 15:85, respectively) is prepared, and the antioxidant is added at a zein:β-carotene proportion of 95:5, respectively. The solutions are kept under stirring until the components are completely dissolved. The final solutions are introduced into a 5-ml syringe in order to subsequently make them pass through the equipment.

Subsequently, in order to perform the coating, the antioxidant is homogeneously deposited and distributed on the collector of the equipment. Finally, the solutions are electrosprayed on the collector. In the case of the coating, following a controlled electrospraying time, the material is tipped and electrosprayed once again in order to ensure complete coating of all the carotene. The equipment is composed of a power supply that supplies between 0 and 30 kV of current, a digital pump where the syringe is placed, and which makes it possible to control the flow rate of the solution, and a stainless steel needle. The needle is connected to the syringe through a teflon tube and placed perpendicular to the collector. In this example, the processing conditions for both materials (coating and encapsulate) were the following: flow rate of the solution of 0.30 ml/h; 12 kV of current; 10-cm distance between the needle and the collector. The non-coated β-carotene, the coated β-carotene and the β-carotene-zein encapsulates were exposed to UV light for 10 h. Table 3 shows the evolution of the absorbance of β-carotene at 455 nm, where this compound presents a characteristic band. The initial absorbance and the absorbance after 10 h under UV light of the non-coated, coated and encapsulated β-carotene are shown. It is observed that coating was the technology that provided the antioxidant with the best protection against UV degradation.

**Table 3**

| | β-carotene | Coating | Encapsulate |
|---|---|---|---|
| Initial absorbance (455 nm) | 0.255 | 0.255 | 0.255 |
| Absorbance 10 h (455 nm) | 0.086 | 0.190 | 0.100 |

### Example 11.

### Coating, in a pilot plant equipment, of the microorganism Bifidobacterium longum subsp. infantis (CECT 4552) from a whey protein concentrate by electrohydrodynamic processing and study of the viability with time under different humidity and temperature conditions.

The first step consists of preparing the solution that forms the nanoparticle coating. To this end, the whey protein concentrate is dissolved at a concentration of 20% by weight with respect to the volume of the solvent (distilled water). Moreover, a surfactant (sorbitan monolaurate) is added, at a concentration of 6% by weight with respect to the weight of the protein, in order to improve the processing. The solution is kept under stirring until the components are completely dissolved. The final solution is introduced into the injection system in order to perform the encapsulation process by means of electrospraying.

In the second place, the lyophilisate is deposited and homogeneously distributed on the collector of the electrohydrodynamic processing equipment.

Subsequently, the solution is electrosprayed on the lyophilisate using a Fluidnatek LE500 equipment from the company Bioinicia S.L., Spain. Following a controlled electrospraying time, the material is tipped and electrosprayed once again, in order to ensure complete coating of the entire lyophilisate. Alternatively, this last step may be performed without tipping the product, which produces the same results, if the collector has been previously electrosprayed with the same whey protein solution concentrate. The equipment is composed of a power supply that supplies between 0 and 30 kV of current in the injector and between 0 and -30kV in the collector, a digital pump where the solution is placed, and which makes it possible to control the flow rate of the latter, and a multi-needle injector.

In this example, the electrospraying conditions were the following: flow rate of the solution of 8 ml/h; 31 kV of potential difference; and 12-cm distance between the injector and the collector. The material collected under these conditions is a coating composed of very fine whey protein concentrate capsules that coat the lyophilisate crystals, as may be observed in Figure 8.

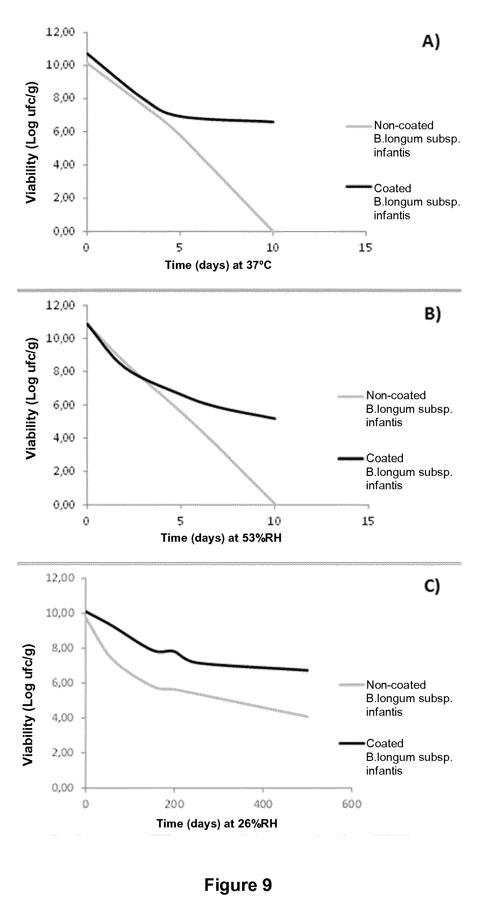

This material was stored jointly with the non-coated lyophilisate at 26% and 53% relative humidity and 37°C, in order to study the viability of the microorganisms. Figure 9 shows the counts of the 2 stored samples (coated and non-coated lyophilisate). It may be observed that, in the case of temperature (37°C) and high humidity (53% RH), the viability of the lyophilisate constantly decreases, to reach zero at 10 days, whereas the viability of the coated lyophilisate becomes stabilised after decreasing for 3-5 days. At 26% RH, the non-encapsulated lyophilised microorganism undergoes a more marked loss of viability than in the case of the lyophilisate encapsulated from the beginning of the study.

## Claims

1. Process for protecting biological material and thermolabile compounds of industrial interest, which comprises the following steps:
- preparing polymer solutions that form micro-, submicro- and nanoparticles which make up coating materials,
- coating the biological material or thermolabile compounds with the materials prepared in the preceding step, **characterised in that** the coating of the biological material or thermolabile compounds is performed by electrohydrodynamic or aerodynamic processing.

2. Process according to claim 1, **characterized in that** the biological materials are microorganisms or viruses.

3. Process according to claim 2, wherein the microorganisms are *Lactobacillus, Bifidobacterium, Cyanobacterium, Rhodobacterales, Saccharomyces* or any other microorganism that requires protection.

4. Process according to claim 1, **characterized in that** the thermolabile compounds are enzymes, vitamins, essential elements or any molecule or compound, whether a derivative or not, that requires protection.

5. Process according to anyone of claims 1 to 4, **characterized in that** the coating materials are selected from proteins, oligosaccharides, polysaccharides, lipids, polymers and combinations thereof.

6. Process according to claim 5, **characterized in that** the coating polymer materials are selected from polyethylene oxide, ethylene-vinyl alcohol copolymers, polyvinyl alcohol, polyvinyl pyrrolidone and combinations thereof.

7. Process according to claim 5, **characterized in that** the proteins used as coating materials are selected from animal, vegetable and microbial proteins, particularly whey proteins, caseins, polypeptides, whether natural or obtained by the genetic modification of microorganisms, collagen, soy protein and zein.

8. Process according to claim 7, **characterized in that** the proteins used as coating materials are selected from zein and whey protein.

9. Process according to claim 5, **characterized in that** the oligosaccharides used as coating materials are selected from lactose, sucrose, maltose and fructo-oligosaccharides, particularly fructo-oligosaccharides.

10. Process according to claim 5, **characterized in that** the polysaccharides used as coating materials are selected from alginate, pectins, chitosan, gums, carrageenans, starch, dextran, maltodextrin, cellulose, glycogen and chitin.

11. Process according to claim 10, **characterized in that** the polysaccharides used as coating materials are selected from dextran, maltodextrin and starch, and any combination thereof.

12. Process according to anyone of claims 1 to 11, **characterized in that** the solvent used to prepare the polymer solutions is selected from water, alcohols, mixtures of alcohols and water, and other organic solvents.

13. Process according to claim 12, **characterized in that** the solvent is water or mixtures of alcohol and water.

14. Process according to anyone of claims 1 to 13, **characterized in that** in the step of preparing the polymer solutions, processing aiding substances are added which facilitate the formation of the micro-, submicro- and nanoparticles, and which are selected from plasticisers, surface active agents, emulsifiers, surfactants, antioxidants or any combination thereof.

15. Process according to claim 14, **characterized in that** the processing aiding substance added is sorbitan monolaurate.

16. Process according to anyone of claims 1 to 15, **characterized in that** the step designed to prepare the polymer solutions includes a homogenisation treatment by stirring.

17. Process according to any of claims 1 to 15, wherein the step of preparing the polymer solutions includes a homogenisation treatment by ultrasounds.

18. Process according to anyone of claims 1 to 17, **characterized in that** the electrohydrodynamic or aerodynamic processing is electrospraying or blow spraying in at least one step.

19. Process according to claim 18, **characterized in that** the electrohydrodynamic or aerodynamic processing is performed in several steps, with a homogenisation, tipping and/or sieving treatment between them.

20. Process according to anyone of claims 1 to 19, **characterized in that** the coating by electrohydrodynamic or aerodynamic processing is directly performed on the biological material or thermolabile compounds, previously dehydrated by means of lyophilisation or another non-electrohydrodynamic or non-aerodynamic process.

21. Process according to anyone of claims 1 to 19, **characterized in that** the electrohydrodynamic or aerodynamic processing is directly performed on the non-dehydrated biological material or thermolabile compounds, occurring the dehydration thereof and after the coating by electrohydrodynamic or aerodynamic processing.

22. Process according to anyone of claims 1 to 21, **characterized in that**, prior to the coating step, a sieving treatment is performed in order to control the particle size.

23. Process according to anyone of claims 1 to 21, **characterized in that**, following the coating step, a sieving treatment is performed in order to control the particle size.

24. Process according to claims 22 or 23, wherein the sieving treatment is mechanical sieving.
